(19) Europäisches Patentamt
European Patent Office
Office européen des brevets

(11) Veröffentlichungsnummer: **0 055 397**
**B1**

# (12) EUROPÄISCHE PATENTSCHRIFT

(45) Veröffentlichungstag der Patentschrift: **22.08.84**

(51) Int. Cl.³: **A 61 K 9/10**, A 61 K 9/70, A 61 K 9/12, A 61 K 31/415

(21) Anmeldenummer: **81109948.0**

(22) Anmeldetag: **27.11.81**

## (54) Antimykotische Mittel mit hoher Wirkstoff-Freisetzung in Form von elastischen Flüssig-Pflastern.

(30) Priorität: **05.12.80 DE 3045914**

(43) Veröffentlichungstag der Anmeldung: **07.07.82 Patentblatt 82/27**

(45) Bekanntmachung des Hinweises auf die Patenterteilung: **22.08.84 Patentblatt 84/34**

(84) Benannte Vertragsstaaten: **AT BE CH DE FR GB IT LI NL SE**

(56) Entgegenhaltungen:
**DD - C - 112 717**
**DE - A - 2 461 406**
**FR - A - 1 589 917**
**FR - A - 2 275 194**
**US - A - 3 476 853**

**CHEMICALS ABSTRACTS, Band 94, Nr. 14. 6. April 1981, Seite 393, Zusammenfassung 109245a, COLUMBUS, OHIO (US)**

(73) Patentinhaber: **BAYER AG, Konzernverwaltung RP Patentabteilung, D-5090 Leverkusen 1 Bayerwerk (DE)**

(72) Erfinder: **von Bittera, Miklos, Max-Scheler-Strasse 7, D-5090 Leverkusen 3 (DE)**
Erfinder: **Büchel, Karl Heinz, Prof. Dr., Dabringhausener Strasse 42, D-5093 Burscheid (DE)**
Erfinder: **Plempel, Manfred, Dr., Pahlkestrasse 5, D-5600 Wuppertal 1 (DE)**
Erfinder: **Regel, Erik, Ing. grad., Bergerheide 72a, D-5600 Wuppertal 1 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft neuartige Formulierungen der bekannten antimykotischen Azolderivate, die eine Depotwirkung trotz Filmbildung und eine höhere Bioverfügbarkeit der Wirkstoffe aufweisen und dadurch eine Kurzeittherapie ermöglichen.

Für die Behandlung von Mykosen beim Menschen, vor allem die Mykosen der Haut, sind bereits Zubereitungen von antimykotischen Derivaten bekannt geworten. Mit diesen Zubereitungen wurden für eine vollständige Sanierung 21 d Therapiezeit benötigt.

Um zu einer Verkürzung der Therapiedauer zu kommen, benötigt man, besonders zur Eliminierung der Keime, bzw. um eine mykologische Sanierung zu erzielen, eine gewisse Depotwirkung und eine höhere Bioverfügbarkeit der Wirkstoffe. Dafür sind die bekannten Formulierungen nur begrenzt geeignet, weil sich von dem vorhandenen Wirkstoffangebot nur ein kleiner Anteil im Flüssigvolumen am Ort der Infektion löst. Wenn man nun ohne weitere Erhöhung der Wirkstoffkonzentration eine Verkürzung der Therapiedauer, z.B. auf 1 d, bei einmaliger Applikation erreichen will, muss man für eine optimale Bioverfügbarkeit des Wirkstoffes Sorge tragen.

Aus FR-A Nr. 2275194, Beispiel G, ist eine Zusammensetzung, die ein Vinylpyrrolidon/Vinylacetat-Copolymer sowie ein antimykotisches Azolderivat enthält, bekannt.

Isopropylmyristat und 2-Octyldodecanol bzw. ein Spreitmittel und ein Lösungsvermittler sind im Zusammenhang mit den oben genannten Copolymeren bekannt aus FR-A Nr. 1589917, Beispiele I bis III.

In diesen beiden Dokumenten findet sich aber keine Angabe, dass die betreffenden Zusammensetzungen die Erzielung einer befriedigenden Wirkung durch eine einmalige Applikation ermöglichen würden.

Es wurde nun gefunden, dass solche Formulierungen antimykotischer Wirkstoffe, die 2 bis 10 Gew.-% Spreitmittel aus der Gruppe bestehend aus Isopropylmyristat, Isopropylpalmitat, Isopropylstearat, Capryl/Caprinsäureester von gesättigten Fettalkoholen der Kettenlänge $C_{12}$ bis $C_{18}$, wachsartige Fettsäureester wie künstliches Entenbürzeldrüsenfett, Silikonöle, Isopropylmyristat/Isopropylpalmitat/Isopropylstearat-Gemisch, Kokosfettsäureisopropylester, 1 bis 8 Gew.-% Lösungsvermittler und als Filmbildner ein schnell trocknendes Polyvinylpyrrolidon, ein Terpolymerisat aus 30% Vinylpyrrolidon, 40% Vinylacetat und 30% Vinylpropionat oder ein Vinylpyrrolidon/Vinylacetat-Copolymerisat, das sowohl in Wasser als auch in organischen Lösungsmitteln löslich ist, und asserdem die üblichen Formulierungshilfsstoffe enthalten, eine optimale Freisetzung des Wirkstoffs und damit eine auf einen Tag verkürzte Therapiedauer durch das Erreichen von hohen Konzentrationen des Wirkstoffes ermöglichen. Dieser Effekt wird dadurch erreicht, dass die Wirkung der in den Formulierungen enthaltenen Wirkstoffe durch Spreitöle und Lösungsvermittler und adhärierende Filmbildnerzugabe erhöht werden und dadurch die Wirkstofffreisetzung bis ins Zehnfache gesteigert werden kann.

Die erfindungsgemässen elastischen Flüssig-Pflaster-Formulierungen stellen ein neues Applikationsprinzip zur dermalen Behandlung von Mykosen dar, das neben einer sehr guten Wirksamkeit durch den Verschluss der Infektionsstelle einen Infektionsschutz für die Umgebung darstellt. Besonders gut geeignet sind die erfindungsgemässen Formulierungen zur Behandlung von Nagelmykosen.

Die erfindungsgemässen Formulierungen können sowohl Lösungen als auch Sprays sein.

Wirkstoffe, die in dieser Weise formuliert werden können, sind alle antimykotisch wirksamen Azolderivate, insbesondere Imidazol- und Triazolderivate. Sie sind in den erfindungsgemässen Mitteln in Mengen von 0,05 bis 1, vorzugsweise 0,1 bis 1 Gew.-%, vorhanden.

Beispielsweise seien die Verbindungen der nachstehenden Formeln genannt:

(I) 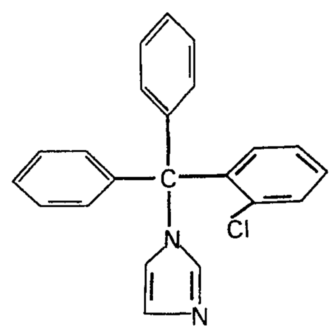 Clotrimazol

(II) 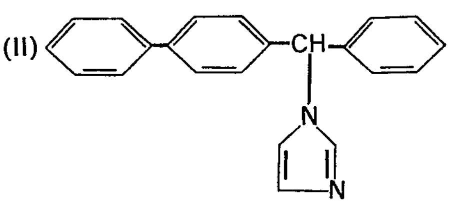 Trifonazol

(III) 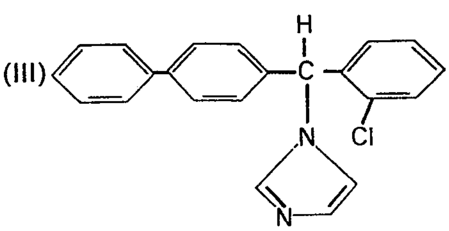 Lombazol

Zahlreiche weitere antimykotisch wirksame Azoldeirvate sind bekannt aus der DE-OS Nr. 2430039. Sie können ebenfalls in den erfindungsgemässen Mitteln als Wirkstoff dienen.

Unter Spreitmitteln werden ölige Flüssigkeiten verstanden, die sich auf der Haut besonders gut verteilen [R. Keymer, „Pharm. Ind.", 32 (1970), 577-581].

Als Lösungsvermittler eignen sich für die erfindungsgemässen Mittel vor allem: Benzylalkohol, 2-Octyldodecanol, Polyethylenglykole, Phthalate, Adipate, Propylenglykol, Glycerin, Di- und Tripropylenglykol, Wachse etc., und andere in der Kosmetik verwendete Zusatzstoffe.

Als Filmbildner kommen solche makromolekularen Verbindungen in Frage, die sich sowohl in Wasser als auch in organischen Lösungsmitteln lösen bzw. anquellen können und nach dem Trocknen eine Art Film bilden. Solche Filmbilder sind Polyvinylpyrrolidon oder Vinylpyrrolidon/Vinylacetat-Copolymerisate mit unterschiedlichem Vinylacetatgehalt. Auch ein Terpolymerisat aus 30 Gew.-% Vinylpyrrolidon, 40 Gew.-% Vinylacetat und 30 Gew.-% Vinylpropionat ist geeignet.

Als Lösungsmittel sind Wasser und auch alle mit Wasser mischbaren Lösungsmittel geeignet. In Betracht kommen z.B. Alkanole, wie Ethanol und Isopropylalkohol, Propylenglykol, Methylcellosolve, Cellosolve, Ester, Morpholine, Dioxan, Dimethylsulfoxid, Dimethylformamid, Tetrahydrofuran, Cyclohexanon etc.

Es können bei der Herstellung der erfindungsgemässen Formulierungen ein oder mehrere Lösungsmittel eingesetzt werden.

Bei den Versuchen zur Ermittelung einer optimalen Formulierung können u.a. folgende Hilfsstoffe eingesetzt werden: Glycerin, Paraffin dickflüssig, Paraffin dünnflüssig, Triethanolamin, Collagen, Allantoin, Novantisolsäure, Parfümöle.

Als weitere Hilfsmittel sind geeignet:

a) Substanzen, die z.B. eine Suspension stabilisieren können, z.B. kolloidale Kieselsäure, Montmorillonite u.a.

b) Tenside (beinhaltet Emulgatoren und Netzmittel), z.B.:

1. anionaktive, wie Na-Laurylsulfat, Fettalkoholethersulfate, Mono/Dialkylpolyglykoletherorthophosphorsäureester/Monoethanolaminsalz;

2. kationaktive, wie Cetyltriemthylammoniumchlorid;

3. ampholytische, wie Di-Na-N-Lauryl-β-iminodipropionat oder Lecithin;

4. nicht ionogene, z.B. polyoxyethyliertes Rizinusöl, polyoxyethyliertes Sorbitanmonooleat, Sorbitanmonostearat, Cetylalkohol, Glycerinmonostearat, Polyoxyethylenstearat, Alkylphenolpolyglykolether.

c) Stabilisatoren zur Verhinderung des bei einigen Wirkstoffen eintretenden chemischen Abbaues wie Antioxydantien, z.B. Tocopherole, Butylhydroxyanisol.

d) Sauer eingestellte wässerige Lösungen können durch den Zusatz in der Kosmetik üblichen Konservierungsmittel, z.B. p-Hydroxybenzoesäureester, stabilisiert werden.

Wirksamkeitstestung der erfindungsgemässen Mittel am trichophytoninfizierten Meerschweinchen.

Als Testmodell zur vergleichenden Wirksamkeitsprüfung der erfindungsgemässen Mittel verwendeten wir trichophytoninfizierte Pirbrightwhite-Meerschweinchen mit einem durchschnittlichen Gewicht von 600 g. Die Tiere wurden auf dem Rücken mit einer elektrischen Haarschneidemaschine so geschoren, dass ca. 1/10 mm lange Haarstümpfe stehen blieben.

Die Infektion mit *Trichophyton mentagrophytes* erfolgte durch leichtes Verreiben einer 24 h in Sabouraud-Nährlösung angekeimten Sporensuspension des Erregers auf einer ca. 2 × 2 cm grossen Fläche des geschorenen Rückens der Tiere. Aufgetragen wurden pro Tier 0,5 ml Keimsuspension, die 1-3 × $10^5$ infektiöse Pilzpartikel enthielten.

Bei diesem Infektionsmodus zeigen sich 2-3 d *post infectionem* die ersten Symptome der Dermatophytose als Rötung und Schuppung der Haut. Bei unbehandelten Tieren ist ca. 14 d *post infectionem* die Dermatophytose maximal ausgeprägt: flächiger Haarausfall und blutige Integument-Defekte innerhalb einer entzündlich veränderten, schuppigen Randzone.

Die zu prüfenden Formulierungen wurden 1 mal, am zweiten Tag *post infectionem*, lokal auf die gerötete Infektionsstelle der Tiere appliziert. Es wurden jeweils 0,5 ml der Formulierungen = 5 mg Wirkstoff* aufgetragen. Die Bewertung des Infektionsablaufs erfolgte täglich bis zum zwanzigsten Tag *post infectionem*.

Die Ergebnisse sind bei den Beispielen angegeben. (+ = schwache Wirkung, ++ = Wirkung, +++ = gute Wirkung, ++++ = sehr gute Wirkung.)

In den nachstehenden Beispielen sind Rezepturen für erfindungsgemässe Mittel angegeben. Die einzelnen Komponenten werden bei Zimmertemperatur miteinander vermischt und gehen dabei in Lösung.

Verwendet man anstelle der erfindungsgemässen Formulierungen solche, die anstelle der genannten Polymere wasserunlösliche Polymere, z.B. Methacrylate, enthalten, so wird die Mykose verschlimmert.

Verwendet man solche Formulierungen, die neben dem Wirkstoff nur die genannten Polymere, aber weder Spreitmittel noch Lösungsvermittler enthalten, erzielt man nur eine schwache Wirkung.

*Beispiel 1:*

| | |
|---|---|
| Trifonazol | 1,000 g |
| Benzylalkohol | 4,000 g |
| Isopropylmyristat | 6,000 g |
| Vinylpyrrolidon/Vinylacetat-Copolymer | 12,500 g |
| Isopropanol | ad 100 ml |

Wirkung im Meerschweinchen-Test +++ = gute Wirkung

*Beispiel 2:*

| | |
|---|---|
| Trifonazol | 1,00 g |
| 2-Octyldodecanol | 2,00 g |
| Isopropylmyristat | 6,00 g |
| Vinylpyrrolidon/Vinylacetat-Copolymer | 10,00 g |
| Isopropanol | ad 100 ml |

Wirkung im Meerschweinchen-Test ++++ = sehr gute Wirkung

*Beispiel 3:*

| | |
|---|---|
| Lombazol | 1,00 g |
| Benzylalkohol | 5,00 g |
| Isopropylstearat | 6,00 g |

* 1%ige Formulierung

Vinylpyrrolidon/Vinylacetat-Copolymer 10,00 g
Isopropanol ad 100 ml
Wirkung im Meerschweinchen-Test ++++ = sehr gute Wirkung

*Beispiel 4:*

| | |
|---|---|
| Lombazol | 0,10 g |
| Benzylalkohol | 6,00 g |
| Isopropylmyristat | 6,00 g |
| Vinylpyrrolidon/Vinylacetat-Copolymer | 10,00 g |
| Isopropanol | ad 100 ml |

Wirkung im Meerschweinchen-Test ++++ = sehr gute Wirkung

*Beispiel 5:*

| | |
|---|---|
| Clotrimazol | 1,00 g |
| Benzylalkohol | 5,00 g |
| Isopropylmyristat | 6,00 g |
| Vinylpyrrolidon/Vinylacetat-Copolymer | 12,50 g |
| Isopropanol | ad 100 ml |

Wirkung im Meerschweinchen-Test ++++ = sehr gute Wirkung

*Beispiel 6:*

| | |
|---|---|
| Clotrimazol | 1,00 g |
| Benzylalkohol | 6,00 g |
| Isopropylmyristat | 6,00 g |
| Vinylpyrrolidon/Vinylacetat-Copolymer | 10,00 g |
| Isopropanol | ad 100 ml |

Wirkung im Meerschweinchen-Test ++++ = sehr gute Wirkung

*Beispiel 7:*

| | |
|---|---|
| Clotrimazol | 1,00 g |
| 2-Octyldodecanol | 2,00 g |
| Isopropylmyrisat | 6,00 g |
| Vinylpyrrolidon/Vinylacetat-Copolymer | 10,00 g |
| Isopropanol | ad 100 ml |

Wirkung im Meerschweinchen-Test ++++ = sehr gute Wirkung

*Beispiel 8:*

| | |
|---|---|
| Clotrimazol | 1,00 g |
| Benzylalkohol | 4,00 g |
| Isopropylmyristat | 6,00 g |
| Vinylpyrrolidon/Vinylacetat-Copolymer | 12,50 g |
| Isopropanol | ad 100 ml |

Wirkung im Meerschweinchen-Test ++++ = sehr gute Wirkung

*Beispiel 9:*

| | |
|---|---|
| Lombazol | 1,00 g |
| 2-Octyldodecanol | 8,00 g |
| Isopropylstearat/Isopropylmyristat/Isopropylpalmitat | 2,00 g |
| Vinylpyrrolidon/Vinylacetat-Copolymer | 10,00 g |
| Isopropanol | ad 100 ml |

Wirkung im Meerschweinchen-Test ++++ = sehr gute Wirkung

*Beispiel 10:*

| | |
|---|---|
| Lombazol | 0,10 g |
| 2-Octyldodecanol | 1,00 g |
| Isopropylmyristat | 10,00 g |
| Vinylpyrrolidon/Vinylacetat-Copolymer | 10,00 g |
| Isopropanol | ad 100 ml |

Wirkung im Meerschweinchen-Test +++ = gute Wirkung

*Beispiel 11:*

| | |
|---|---|
| Clotrimazol | 1,0 g |
| Benzylalkohol | 5,0 g |
| Isopropylmyristat | 6,0 g |
| Polyvinylpyrrolidon | 12,5 g |
| Isopropanol | ad 100 ml |

Wirkung im Meerschweinchen-Test ++++ = sehr gute Wirkung

*Beispiel 12:*

| | |
|---|---|
| Lombazol | 1,0 g |
| Benzylalkohol | 5,0 g |
| Isopropylmyristat/Isopropylpalmitat/Isopropylstearat-Gemisch | 6,0 g |
| Polyvinylpyrrolidon | 12,5 g |
| Isopropanol | ad 100 ml |

Wirkung im Meerschweinchen-Test ++++ = sehr gute Wirkung

*Beispiel 13:*

| | |
|---|---|
| Clotrimazol | 1,0 g |
| Benzylalkohol | 2,0 g |
| Isopropylmyristat | 6,0 g |
| Terpolymerisat aus 30% Vinylpyrrolidon, 40% Vinylacetat und 30% Vinylpropionat | 12,5 g |
| Isopropanol | ad 100 ml |

Wirkung im Meerschweinchen-Test ++++ = sehr gute Wirkung

*Beispiel 14:*

| | |
|---|---|
| Lombazol | 1,0 g |
| Benzylalkohol | 5,0 g |
| Gemisch aus Isopropylmyristat/Isopropylstearat und Isopropylpalmitat | 6,0 g |
| Terpolymerisat aus 30% Vinylpyrrolidon, 40% Vinylacetat und 30% Vinylpropionat | 12,5 g |
| Isopropanol | ad 100 ml |

Wirkung im Meerschweinchen-Test ++++ = sehr gute Wirkung

*Sprays*

Die nach den Beispielen 1 bis 14 hergestellten Wirkstofflösungen können auch zu Sprays verarbeitet werden. Zu diesem Zweck vermischt man z.B. 60-90% Wirkstofflösung mit 20-40% der gebräuchlichen Treibmittel, z.B. $N_2$, $N_2O$, $CO_2$, Propan, Butan, Halogenkohlenwasserstoffe etc.

**Patentansprüche** für die Vertragsstaaten: BE, CH, LI, DE, FR, GB, IT, NL, SE

1. Antimykotische Mittel mit höherer Freisetzung der Wirkstoffe, enthaltend Azolderivate und übliche Formulierungshilfsstoffe, dadurch gekennzeichnet, dass sie 2 bis 10 Gew.-% Spreitmittel aus der Gruppe bestehend aus Isopropylmyristat, Isopropylpalmitat, Isopropylstearat, Capryl/Caprinsäurester von gesättigten Fettalkoholen der Kettenlänge $C_{12}$ bis $C_{18}$, wachsartige Fettsäureester wie künstliches Entenbürzeldrüsenfett, Silikonöle, Isopropylmyristat/Isopropylpalmitat/Isopropylstearat-Gemisch, Kokosfettsäureisopropylester, 1 bis 8 Gew.-% Lösungsvermittler und als Filmbildner ein schnell trocknendes Polyvinylpyrrolidon, ein Terpolymerisat aus 30 Gew.-% Vinylpyrrolidon, 40 Gew.-% Vinylacetat und 30 Gew.-% Vinylpropionat oder ein Vinylpyrrolidon/Vinylacetat-Copolymer enthalten.

2. Antimykotische Mittel nach Anspruch 1, dadurch gekennzeichnet, dass sie als Wirkstoff Clotrimazol der Formel

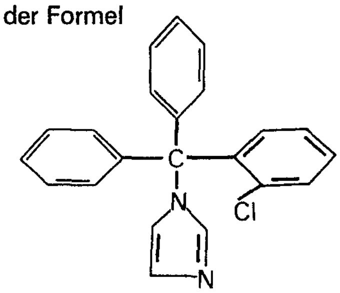

enthalten.

3. Antimykotische Mittel nach Anspruch 1, dadurch gekennzeichnet, dass sie als Wirkstoff Trifonazol der Formel

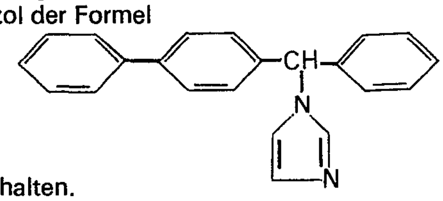

enthalten.

4. Antimykotische Mittel nach Anspruch 1, dadurch gekennzeichnet, dass sie als Wirkstoff Lombazol der Formel

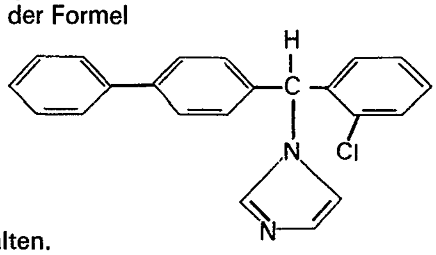

enthalten.

5. Antimykotische Mittel nach Anspruch 1, dadurch gekennzeichnet, dass sie die antimykotischen Azolderivate in Mengen von 0,05 bis 1, vorzugsweise von 0,1 bis 1 Gew.-%, enthalten.

6. Antimykotisches Mittel nach Anspruch 1, dadurch gekennzeichnet, dass es eine Lösung ist.

7. Antimykotisches Mittel nach Anspruch 1, dadurch gekennzeichnet, dass es ein Spray ist.

**Patentansprüche** für den Vertragstaat: AT

1. Verfahren zur Herstellung von Antimykotika auf Basis von Azolderivatwirkstoffen und üblichen Formulierungshilfsstoffen, dadurch gekennzeichnet, dass die Wirkstoffe und Formulierungshilfsstoffe zusammen mit 2 bis 10% Masse Isopropylmyristat, Isopropylpalmitat, Isopropylstearat, Capryl/Caprinsäureester von gesättigten Fettalkoholen der Kettenlänge $C_{12}$ bis $C_{18}$, wachsartige Fettsäureester wie künstliches Entenbürzeldrüsenfett, Silikonöle, Isopropylmyristat/Isopropylpalmitat/Isopropylstearat-Gemisch oder Kokosfettsäureisopropylester als Spreitmittel, mit 1 bis 8% Masse Lösungsvermittler, jeweils bezogen auf die Gesamtmasse, und mit einem schnell trocknenden Polyvinylpyrrolidon, einem Terpolymer aus Vinylpyrrolidon/Vinylacetat/Vinylpropionat im Masseverhältnis 30:40:30, oder einem Vinylpyrrolidon/Vinylacetat-Copolymer als Filmbildner, formuliert werden.

2. Verfahren nach Anspruch 1, dadurch gekennzeichnet, dass die Wirkstoffe, insbesondere Clotrimazol, Trifonazol oder Lombazol, in Mengen von 0,05 bis 1, vorzugsweise von 0,1 bis 1% Masse, bezogen auf die Gesamtmasse, eingesetzt werden.

3. Verfahren nach einem der beiden Ansprüche 1 oder 2, dadurch gekennzeichnet, dass ein Antimykotikum in Form einer Lösung formuliert wird.

4. Verfahren nach Anspruch 3, dadurch gekennzeichnet, dass eine Lösung zur Anwendung als Spray formuliert wird.

**Revendications** pour les Etats contractants: BE, CH, LI, DE, FR, GB, IT, NL, SE

1. Produits antimycotiques ou antifongiques présentant une plus grande libération des substances actives, contenant des dérivés de l'azole et des adjuvants usuels de formulation, produits caractérisés en ce qu'ils contiennent 2 à 10% en poids d'un agent d'étalement choisi dans l'ensemble, constitué par le myristate d'isopropyle, le palmitate d'isopropyle, le stéarate d'isopropyle, des esters de l'acide caprylique/caprique d'alcools gras saturés ayant une longueur de chaîne correspondant à $C_{12}$ à $C_{18}$, des esters d'acides gras cireux comme de la matière grasse de glandes de croupion de canard, des huiles de silicone, du mélange myristate d'isopropyle/palmitate d'isopropyle/stéarate d'isopropyle, de l'ester isopropylique gras de noix de coco, 1 à 8% en poids d'un tiers solvant, et comme filmogène une polyvinylpyrrolidone à séchage rapide, un terpolymère de 30% en poids de vinylpyrrolidone, 40% en poids d'acétate de vinyle et 30% en poids de propionate de vinyle ou un copolymère vinylpyrrolidone/acétate de vinyle.

2. Produits antimycotiques selon la revendication 1, caractérisés en ce qu'ils contiennent comme substance active du clotrimazol de formule:

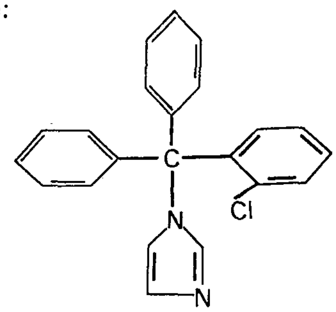

3. Produits antimycotiques selon la revendication 1, caractérisés en ce qu'ils contiennent comme substance active du Trifonazol de formule:

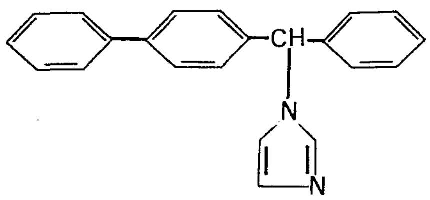

4. Produits antimycotiques selon la revendication 1, caractérisés en ce qu'ils contiennent comme substance active du Lombazol de formule:

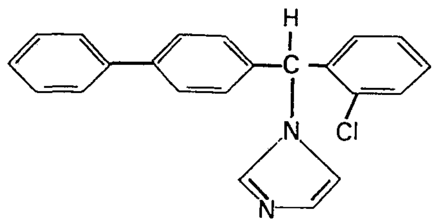

5. Produits antimycotiques selon la revendication 1, caractérisés en ce qu'ils contiennent les dérivés antimycotiques de l'azole en des quantités de 0,05 à 1% en poids, avantageusement de 0,1 à 1% en poids.

6. Produit antimycotique selon la revendication 1, caractérisé en ce qu'il est une solution.

7. Produit antimycotique selon la revendication 1, caractérisé en ce qu'il est un produit à pulvériser.

**Revendications** pour Etat contractant: AT

1. Procédé pour la fabrication d'antimycosiques à base de dérivés azoles comme matières actives et des auxiliaires de formulation habituels, caractérisé par le fait que les matières actives et les auxiliaires de formulation sont formulés avec 2 à 10% en masse d'isopropylmyristate, d'isopropylpalmitate, d'isopropylstéarate, d'esters capryl/capriniques d'alcools gras saturés de longueur de chaîne $C_{12}$-$C_{18}$, d'esters d'acides gras de caractère céroïde, tels que la matière sébacée de la glande uropygienne du canard produits artificiellement, d'huiles de silicones, d'un mélange isopropylmyristate/isopropylpalmitate/isopropylstéarate ou d'ester isopropylique d'acide gras de coco excipient, avec 1 à 8% en masse de solubilisant, ces proportions étant calculées chaque fois par rapport à la masse totale, et avec un polyvinylpyrrolidone à séchage rapide, un terpolymère de vinylpyrrolidone/vinylacétate/vinylproprionate dans le rapport massique 30:40:30, ou un copolymère de vinylpyrrolidone/vinylacétate comme agent filmogène.

2. Procédé suivant la revendication 1, caractérisé par le fait que les matières actives, en particulier clotrimazole, trifonazole ou lombazole, sont mises en œuvre à raison de 0,5 à 1% en masse, principalement de 0,1 à 1% en masse, ces proportions étant calculées par rapport à la masse totale.

3. Procédé suivant l'une des revendications 1 ou 2, caractérisé par le fait qu'un antimycosique est formulé sous forme de solution.

4. Procédé suivant la revendication 3, caractérisé par le fait qu'une solution est formulée pour être appliquée sous forme de spray.

**Claims** for the contracting states: BE, CH, LI, DE, FR, GB, IT, NL, SE

1. Antimycotic agents which have relatively good release of active compounds and contain azole derivatives and customary formulation auxiliaries, characterised in that they contain 2-10% by weight of a spreading agent from the group consisting of isopropyl myristate, isopropyl palmitate, isopropyl stearate, caprylates/caproates of saturated fatty alcohols having the chain length of $C_{12}$ to $C_{18}$, wax-like fatty acid esters, such as synthesized duck uropygial gland fat, silicone oils, isopropyl myristate/isorpopyl palmitate/isopropyl stearate mixture, coconut fatty acid isopropyl ester, 1-8% by weight of a solubilising agent and, as a film-forming agent, a quick-drying polyvinylpyrrolidone, a terpolymer of 30% by weight of vinylpyrrolidone, 40% by weight of vinyl acetate and 30% by weight of vinyl propionate, or a vinylpyrrolidone/vinyl acetate copolymer.

2. Antimycotic agents according to Claim 1, characterised in that they contain clotrimazole, of the formula

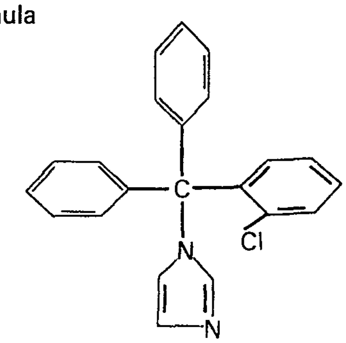

as the active compound

3. Antimycotic agents according to Claim 1, characterised in that they contain trifonazole, of the formula

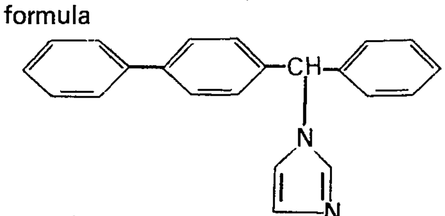

as the active compound.

4. Antimycotic agents according to Claim 1, characterised in that they contain lombazole, of the formula

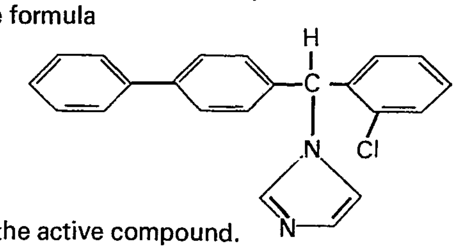

as the active compound.

5. Antimycotic agents according to Claim 1, characterised in that they contain the antimycotic azole derivatives in amounts of 0.05 to 1% by weight, preferably 0.1 to 1% by weight.

6. Antimycotic agent according to Claim 1, characterised in that it is a solution.

7. Antimycotic agent according to Claim 1, characterised in that it is a spray.

**Claims** for the contracting state: AT

1. Process for the production of antimycotics based on azole derivative active compounds and costumary formulation auxiliaries, characterised in that the active compounds and the formulation auxiliaries are formulated together with 2 to 10% by weight of isopropyl myristate, isopropylpalmitate, isopropyl stearate, caprylates/caproates of saturated fatty alcohols having the chain length of $C_{12}$ to $C_{18}$, waxy fatty acid esters, such as synthesized duck uropygial gland fat, silicone oils, isopropyl myristate/isopropyl palmitate/isopropyl stearate mixture or cocount fatty acid isopropylester as spreading agents, 1 to 8% by weight of solubilising agents, based in each case on the total weight, and a quick drying polyvinylpyrrolidone, a terpolymer of vinylpyrrolidone/vinyl acetate/vinyl propionate in a ratio by weight of 30:40:30, or a vinylpyrrolidone/vinylacetate copolymer as the film-forming agent.

2. Process according to Claim 1, characterised in that the active compounds, in particular clotrimazole, trifonazole, or lombazole, are used in quantities of 0.05 to 1% by weight, preferably 0.1 to 1% by weight, based on the total weight.

3. Process according to Claims 1 or 2, characterised in that an antimycotic is formulated in the form of a solution.

4. Process according to Claim 3, characterized in that a solution is formulated for use as a spray.